Europäisches Patentamt

**European Patent Office**

**Office européen des brevets**

(19)

(11) Publication number: **0 113 569**
A2

(12) # EUROPEAN PATENT APPLICATION

(21) Application number: **83307680.5**

(22) Date of filing: **16.12.83**

(51) Int. Cl.³: **C 12 P 13/14**
C 12 N 15/00, C 12 N 1/20
//C12R1/13, C12R1/15

A request for correction under Rule 88 E.P.C. by subsequent filing of missing page 3 has been received on 14.02.1984.

(30) Priority: **17.12.82 JP 221381/82**

(43) Date of publication of application:
**18.07.84 Bulletin 84/29**

(84) Designated Contracting States:
**DE FR GB IT**

(71) Applicant: **KYOWA HAKKO KOGYO CO., LTD.**
**Ohtemachi Bldg., 6-1 Ohtemachi Itchome**
**Chiyoda-ku Tokyo 100(JP)**

(72) Inventor: **Nakanishi, Toshidide**
**2-3-205, Kyowa-cho**
**Hofu-shi Yamaguchi-ken(JP)**

(72) Inventor: **Yonekura, Hideaki**
**2-10-104, Kyowa-cho**
**Hofu-shi Yamaguchi-ken(JP)**

(72) Inventor: **Azuma, Tomoki**
**2-10-106, Kyowa-cho**
**Hofu-shi Yamaguchi-ken(JP)**

(72) Inventor: **Hattori, Kiyoji**
**2-12-106, Kyowa-cho**
**Hofu-shi Yamaguchi-ken(JP)**

(74) Representative: **Lambert, Hugh Richmond et al,**
**D. YOUNG & CO. 10 Staple Inn**
**London, WC1V 7RD(GB)**

(54) **Process for producing L-glutamic acid by fermentation.**

(57) A process is disclosed for producing L-glutamic acid, the process involves culturing a microorganism having an ability to produce L-glutamic acid in a nutrient medium until L-glutamic acid is accumulated in the culture liquor and recovering the L-glutamic acid therefrom. The process is characterized by using a mutant obtained by the technology of protoplast fusion or a mutant having a resistance to two or more antibiotics. Also disclosed are novel microorganisms for use therein.

EP 0 113 569 A2

Croydon Printing Company Ltd.

- 1 -

PROCESS FOR PRODUCING L-GLUTAMIC
ACID BY FERMENTATION
AND NOVEL MICROORGANISM STRAINS
FOR USE THEREIN

The present invention relates to a process for producing L-glutamic acid by fermentation and microorganism, for use therein.

L-glutamic acid is an important amino acid which is commercially useful as a food additive.

Heretofore, L-glutamic acid has been produced by fermentation processes using microorganism strains of the genus Corynebacterium or Brevibacterium. The production yields of these known processes are comparatively low and a need exists for processes for producing L-glutamic acid at higher yields and at low cost.

In accordance with the present invention it has been found that L-glutamic acid can be produced in high yield utilising microorganisms belonging to the genus Corynebacterium or Brevibacterium and which have either been endowed with a resistance to two or more antibiotics, and/or obtained by protoplast fusion between parent strains belonging to the same or different genus of Corynebacterium or Brevibacterium.

Also included herein are novel microorganism strains of Corynebacterium and Brevibacterium being stains obtained by protoplast fusion and having an ability to produce L-glutamic acid, and desirably also having a resistance to two or more antibiotics.

As a microorganism strain having both an ability to produce L-glutamic acid and a resistance to two or more antibiotics, there may be used either a strain of Corynebacterium or Brevibacterium which is inherently capable of producing L-glutamic acid and which has been additionally endowed with a resistance to two or more antibiotics, or a strain of Corynebacterium or Brevibacterium which inherently has the specified resistance and which has been additionally endowed with an ability of producing L-glutamic acid.

Examples of antibiotics to which the strain is resistant include aminoglycoside type antibiotics such as streptomycin, dihydrostreptomycin, kanamycin, kasugamycin, gentamicin, sagamicin, fortimicin, etc., B-lactam type antibiotics such as cephalosporins, etc., macrolide type antibiotics such as spiramycin, erythromycin, oleandomycin, lincomycin, etc., rifamycin type antibiotics such as rifampicin, peptide type antibiotics such as bacitracin, gramicidin, polymixin, colistin, thiocillin, bacilysin, polcillin, brevolin, surfactin, circulin, tyrocidin and subtilysin.

Parent strains to be used for obtaining mutants useful in the present invention are microorganisms belonging to the genus Corynebacterium or Brevibacterium known as a glutamic acid- (hereinafter referred to as H-3333)(FERM BP-415) and Brevibacterium lactofermentum H-3361 (hereinafter referred to as H-3361) (FERM BP-419). Specific examples of a strain having an ability to produce L-glutamic acid obtained by the technology of protoplast fusion are a protoplast fusion strain H-3359 (hereinafter referred to as H-3359) (FERM BP-417) between H-3332 and H-3339. The mutants H-3332, H-3333, H-3339, H-3359, H-3360, H-3361 and H-3362 were deposited on November 27, 1982 with the Fermentation Research Institute, the Agency of Industrial Science and Technology, Japan under FERM P numbers shown below.

The strains were transferred to a deposit under the Budapest Treaty on December 1, 1983, and the corresponding international deposit numbers are shown below.

| Strain | FERM P No. | FERM BP No. |
|--------|------------|-------------|
| H-3332 | 6799 | 414 |
| H-3333 | 6800 | 415 |
| H-3339 | 6801 | 416 |
| H-3359 | 6802 | 417 |
| H-3360 | 6803 | 418 |
| H-3361 | 6804 | 419 |
| H-3362 | 6805 | 427 |

An example of obtaining a protoplast fusion strain of the present invention is described below.

Protoplasts are formed from culture cells as follows. A strain is inoculated in a nutrient medium NB containing 20 g of bouillon powder and 5 g of yeast extract in 1 ℓ of pure water and adjusted to pH 7.2, and cultured with shaking. Absorbance (OD) at 660 nm is measured by means of a turbidimeter and, in the initial stage (OD = 0.1 to 0.2) of logarithmic growth phase, penicillin G is added to the culture liquor to make a final concentration of 0.1 to 0.8 μg/ml. Culturing is further continued and, when OD reaches 0.3 to 0.5, cells are collected and washed with SSM medium (containing 10 g of glucose, 4 g of $NH_4Cl$, 2 g of urea, 1 g of yeast extract, 1 g of $KH_2PO_4$, 3 g of $K_2HPO_4$, 0.4 g of $MgCl_2 \cdot 6H_2O$, 10 mg of $FeSO_4 \cdot 7H_2O$, 0.2 mg of $MnSO_4 \cdot 4-6H_2O$, 0.9 mg of $ZnSO_4 \cdot 7H_2O$, 0.4 mg of $CuSO_4 \cdot 5H_2O$, 0.09 mg of $Na_2B_4O_7 \cdot 10H_2O$, 0.04 mg of $(NH_4)_6Mo_7O_{24} \cdot 4H_2O$, 30 μg of biotin and 1 mg of thiamine hydrochloride in 1 ℓ of pure water and adjusted to pH 7.2; in culturing an amino acid-requiring strain, 50 μg/ml of a required amino acid is further added SSM medium). Then, the cells are again suspended in PFM medium (containing 0.4 M sucrose and 0.01 M $MgCl_2 \cdot 6H_2O$ in a 2-fold diluted SSM solution and adjusted to pH 7.0 to 8.5). Lysozyme (0.2 to 2 mg/ml) is added to this cell suspension to keep at 30 to 37°C for 16 hours. Formation of protoplast is confirmed under an optical microscope. The thus prepared protoplasts of two strains to be fused are counted under an optical microscope and the suspensions are mixed in a protoplast ratio of 1:1. The protoplasts are separated from the mixture by centrifugation and after washing the separated protoplasts with PFM medium, the protoplasts are again suspended in 0.1 ml of PFM medium.

To the suspension is added 2.5 ml of PFM medium containing 40% polyethylene glycol (PEG) 4000, and slowly stirred for 5 minutes.

In the case where a streptomycin-resistant strain and a rifampicin-resistant strain are used, 0.3 ml of the suspension is smeared on RCG agar plate containing 100 μg/ml of streptomycin and 0.05 μg/ml of rifampicin (containing 5 g of glucose, 5 g of casamino acid, 2.5 g of yeast extract, 3.5 g of $K_2HPO_4$, 1.5 g of $KH_2PO_4$, 0.41 g of $MgCl_2 \cdot 6H_2O$, 10 mg of $FeSO_4 \cdot 7H_2O$, 2 mg of $MnSO_4 \cdot 4-6H_2O$, 0.9 mg of $ZnSO_4 \cdot 7H_2O$, 0.4 mg of $CuSO_4 \cdot 5H_2O$, 0.09 mg of $Na_2B_4O_7 \cdot 10H_2O$, 0.04 mg of $(NH_4)_6Mo_7O_{24} \cdot 4H_2O$, 30 μg of biotin, 2 mg of thiamine hydrochloride, 135 g of sodium succinate and 14 g of agar in 1 ℓ of pure water, and adjusted to pH 7.4). After culturing at 30°C for 12 days, colonies of a strain having a resistance to both streptomycin and rifampicin are obtained.

Any of synthetic medium and natural medium may be used as the medium for the present invention, so long as it properly contains a carbon source, nitrogen source, inorganic materials and other necessary nutrients which are assimilable by the strain utilized.

As the carbon source, various carbohydrates, such as glucose, fructose, sorbitol, glycerol, sucrose, starch, starch hydrolyzate, molasses, fruit juice, etc., organic acids, such as acetic acid, fumaric acid, lactic acid, etc., and alcohols, such as ethanol, methanol, etc., may be used.

As the nitrogen source, ammonia, inorganic and organic ammonium salts, such as ammonium chloride, ammonium sulfate, ammonium acetate, ammonium phosphate, etc., urea, amines, other nitrogen-containing compounds such as peptone, meat extract, yeast extract, corn steep liquor, casein hydrolyzate, acid hydrolyzate of soybean meal, various microbial cells, digest of microbial cells, etc., may be used.

As the inorganic materials, potassium dihydrogen phosphate, dipotassium hydrogen phosphate, magnesium phosphate, magnesium sulfate, sodium chloride, ferrous sulfate, manganese sulfate, copper sulfate, calcium carbonate, etc., are used. When a microorganism to be used in the present invention requires specific nutrients for growth, an appropriate amount of the nutrients are added to the medium. In some cases, these nutrients are added as components of the natural substances exemplified as the nitrogen source.

Further, the productivity of L-glutamic acid by the present microorganism can be, in some cases, enhanced by adding other various additives, for example, various antibiotics such as streptomycin, penicillin G and rifampicin, antioxidant such as α-tocopherol, surfactants such as polyoxyethylene sorbitan-mono-parmitate, amino acids such as methionine, lysine, cysteine and aspartic acid, biotin, acetic acid, oleic acid, adenine, etc., to the medium.

Culturing is carried out under aerobic conditions, for example, by shaking culture, agitation submerged culture, etc. The temperature for culturing is generally 20 - 40°C, and the pH of the medium is in a range of 3 to 9, and is preferably maintained at around neutral, but culturing can be carried out under conditions which are out of this range so long as the microorganism used can grow. The pH of the medium is adjusted with calcium carbonate, acid or alkali solution, ammonia, pH buffering agent, etc. Usually, after culturing for 1 to 4 days, L-glutamic acid is formed and accumulated in the resulting culture liquor.

After the completion of culturing, precipitates, such as cells, are removed from the culture liquor and L-glutamic acid can be recovered from the culture liquor by use of the conventional methods, such as ion-exchange resin treatment, concentration, adsorption, and salting-out in combination.

Practice of specific embodiments of the invention is illustrated by the following representative examples.

Example 1

(1) Preparation of H-3332

Corynebacterium glutamicum H-3283, FERM P-6576 capable of producing L-glutamic acid and having a resistance to 6-azauracil is cultured overnight in a bouillon medium.

The culture is suspended in a 0.1 N tris-maleate buffer solution (pH 6.0) in a concentration of $10^8$ cells/ml. To the suspension is added N-methyl-N'-nitro-N-nitrosoguanidine to make a final concentration of 0.2 mg/ml. The suspension is allowed to stand for 30 minutes at a temperature of 30°C, and is smeared on a bouillon agar plate medium containing 200 μg/ml dihydrostreptomycin. H-3332 is obtained as the mutant selected in the colonies incubated at 30°C for 6 days.

(2) Preparation of H-3360 and H-3362

As a parent strain, H-3332 is used. The same procedures as described in (1) above are repeated except that 5 μg/ml bacitracin is used instead of dihydrostreptomycin to obtain H-3360.

The same procedures as described in (1) above are repeated except that 10 µg/ml gramicidin is used instead of dihydrostreptomycin to obtain H-3362.

(3) Preparation of H-3361

The same procedures as described in (1) above are repeated except that Brevibacterium lactofermentum H-3284 (FERM P-6577) (hereinafter referred to as H-3284) capable of producing L-glutamic acid and having a resistance to 6-mercaptoguanosine is used instead of H-3283 to obtain H-3361.

(4) Preparation of H-3333

The same procedures as described in (1) above are repeated except that H-3361 and rifampicin are used instead of H-3283 and dihydrostreptomycin to obtain H-3333.

Table 1 shows drug-resistance of H-3283, H-3332, H-3360, H-3362, H-3284, H-3361 and H-3333.

Table 1

|  |  | H-3283 | H-3332 | H-3360 | H-3362 | H-3284 | H-3361 | H-3333 |
|---|---|---|---|---|---|---|---|---|
| No addition |  | 100 | 100 | 100 | 100 | 100 | 100 | 100 |
| Dihydro-streptomycin | 100 µg/ml | 14 | 98 | 95 | 97 | 12 | 100 | 100 |
|  | 200 | 10 | 96 | 95 | 95 | 7 | 98 | 96 |
| Rifampicin | 20 | 17 | 16 | 16 | 17 | 15 | 18 | 97 |
|  | 50 | 12 | 13 | 13 | 13 | 10 | 10 | 94 |
| Bacitracin | 2 | 17 | 18 | 15 | 97 | – | – | – |
|  | 5 | 11 | 12 | 13 | 89 | – | – | – |
| Gramicidin | 5 | 15 | 14 | 96 | 20 | – | – | – |
|  | 10 | 10 | 12 | 92 | 15 | – | – | – |

Drug-resistance is indicated by relative values when the growth without addition of a drug is defined as 100.

(5)  Preparation of H-3359

Protoplasts of H-3332 having a resistance to 6-azauracil and dihydrostreptomycin and H-3339 (having a temperature sensitivity remediable with oleic acid) derived from <u>Corynebacterium glutamicum</u> H-2874 (NRRL B-12304) having a temperature sensitivity remediable with oleic acid are prepared, and protoplast fusion is conducted in the same manner as described hereinbefore to obtain a number of fusion strains having a resistance to both rifampicin and dihydrostreptomycin.  With respect to the parent strains, the appearance frequencies of the spontaneous mutant strains having a resistance to both rifampicin and dihydrostreptomycin are $1.1 \times 10^{-8}$ and $3.9 \times 10^{-9}$, respectively;  whereas that by the technology of protoplast fusion is $2.5 \times 10^{-7}$. Accordingly, protoplast fusion endows the parent strains with the double resistance at least 20 times as much as spontaneous mutation.  Thus, it is confirmed that strains having a resistance to both rifampicin and dihydrostreptomycin appear with high frequency by the technology of the protoplast fusion.

Various properties of H-3359, selected as a typical example of the protoplast fusion strain, are shown in Table 2 in comparison with those of parent strains.  As is clear from the table, H-3359 has the same properties as H-3332 with respect to resistance to 6-azauracil and dihydrostreptomycin, and has the same properties as H-3339 with respect to temperature sensitivity remediable with oleic acid and resistance to rifampicin.

Table  2

| | 6-Azauracil | Oleic acid | Dihydro-streptomycin | Rifampicin |
|---|---|---|---|---|
| H - 3332 | R | + | R | S |
| 3339 | S | ts | S | R |
| 3359 | R | ts | R | S |

R :   Resistance

S :   Sensitivity

+ :   No requirement

ts :   Temperature sensitivity

Example 2

        As a seed strain, H-3332 is used.  The strain is
cultured in a seed medium having the composition of 50 g/l
glucose, 5 g/l $(NH_4)_2SO_4$, 1 g/l $KH_2PO_4$, 0.5 g/l $MgSO_4 \cdot 7H_2O$,
200 µg/ml thiamine HCl, 5 g/l urea, 10 mg/l $FeSO_4 \cdot 7H_2O$,
10 mg/l $MnSO_4 \cdot 4H_2O$, 1 mg/l $CuSO_4 \cdot 4H_2O$, and 100 µg/l biotin
(pH 7.0).

        0.5 ml of the seed culture is inoculated into
20 ml of a fermentation medium in a 250 ml-Erlenmeyer flask
sterilized in advance by heating at 120°C for 10 minutes.
The fermentation medium has the same composition as the
seed medium except that 3 µg/l of biotin is used.

        Culturing is carried out at 30°C with shaking
while keeping the pH of the culture liquor at 6.5 - 8.0 with
sterilized urea solution.

        The yield of L-glutamic acid accumulated in the
culture is 26.0 g/l.

        As a control, H-3283 is cultured in the same manner
as described above to obtain L-glutamic acid in the yield
of 24.5 g/l.

Example 3

        The same procedures as described in Example 2 are
repeated except that H-3284, H-3333 and H-3362 are used
instead of H-3332 to obtain L-glutamic acid in the yield of
24.5, 25.5 and 27.0 g/l respectively.

Example 4

        The strains H-3283, H-3332, H-3360 and H-3362 are
used as seed strains.  A fermentation medium having the
composition of 60 g/l blackstrap molasses (as glucose),
5 g/l $(NH_4)_2SO_4$, 5 g/l urea, 1 g/l $KH_2PO_4$, 1 g/l $K_2HPO_4$,

and 20 mg/1 $MnSO_4 \cdot 4H_2O$ (pH 6.5) is prepared. 16 ml of the medium is poured into a 250 ml-Erlenmeyer flask and sterilized at 120°C for 10 minutes. 3 ml of the seed culture obtained by culturing the strains in a medium having the same composition as the fermentation medium is inoculated into the fermentation medium in the 250 ml flask and penicillin G solution is added to a final concentration of 5 U/ml. Culturing is carried out at 34°C with shaking for 30 hours while keeping the pH of the culture liquor at 6.5 - 8.0 with 10% urea solution.

The yield of L-glutamic acid is 33.0 g/1 for H-3283, 34.0 g/1 for H-3332, 35.5 g/1 for H-3360 and 36.0 g/1 for H-3362.

Example 5

In this example, H-3284, H-3361 and H-3333 are used as seed strains.

The strains are cultured in the seed culture medium having the same composition as the seed medium in Example 4.

3 ml of the seed culture is inoculated into 16 ml of a medium prepared by adding 0.08 g/1 polyoxyethylene-sobitan-mono-parmitate to the same fermentation medium as in Example 4.

Culturing is carried out at 34°C for 30 hours while keeping the pH of the culture liquor at 6.5 - 8.0 with 10% sterilized urea solution.

The yield of L-glutamic acid is 29.5 g/1 for H-3284, 30.0 g/1 for H-3361 and 32.0 g/1 for H-3333.

Example 6

The strains listed in Table 3 are inoculated in a seed medium having the composition of 50 g/1 glucose, 10 g/1 peptone, 5 g/1 yeast extract, 5 g/1 meat extract, 5 g/1 ammonium sulfate, 1 g/1 potassium dihydrogen phosphate, 1 g/1 dipotassium monohydrogen phosphate, 0.5 g/1 $MgSO_4 \cdot 7H_2O$, 20 mg/1 $FeSO_4 \cdot 7H_2O$, 20 mg/1 $MnSO_4 \cdot 4H_2O$, 50 µg/1 biotin and 5 g/1 urea (pH 7.2 before sterilization) and cultured at 30°C for 16 hours.  4 ml of the seed culture is added to 20 ml of a medium having the composition of 60 g/l glucose, 5 g/l urea, 5 g/l ammonium sulfate, 1 g/l potassium dihydrogen phosphate, 1 g/l dipotassium monohydrogen phosphate, 20 mg/l $MnSO_4 \cdot 4H_2O$, 100 µg/l biotin, 10 mg/l phenol red and oleic acid in the concentration shown in Table 3  (pH 6.5 before sterilization) in a 250 ml-Erlenmeyer flask, and cultured at 28 or 38°C for 30 hours. During the culturing, 1 ml of 10% urea solution is added when the pH of the culture liquor becomes about 7 as judged by the color of phenol red.

Table 3 shows the result.

## Table 3

| Strain | | H-3332 | H-3339 | | H-3359 | |
|---|---|---|---|---|---|---|
| Amount of sodium oleate (mg/l) | | 0 | 0 | 200 | 0 | 200 |
| 28°C | O D | 0.620 | 0.500 | 0.480 | 0.510 | 0.480 |
| | Yield of L-glutamic acid (g/l) | 0.5 | 1.2 | 1.5 | 1.0 | 2.0 |
| 38°C | O D | 0.410 | 0.230 | 0.400 | 0.240 | 0.410 |
| | Yield of L-glutamic acid (g/l) | 8.0 | 27.0 | 11.0 | 31.5 | 8.5 |

CLAIMS

1. A process for the production of L-glutamic acid which comprises culturing an L-glutamic acid producing microorganism of the genus Corynebacterium or Brevibacterium in a culture medium, allowing the L-glutamic acid to accumulate in the culture medium and recovering the accumulated L-glutamic acid characterised in that the microorganism used is a strain of Corynebacterium or Brevibacterium which has, or has been endowed with, a resistance to two or more antibiotics and/or which has been obtained by protoplast fusion between two different strains of Corynebacterium or Brevibacterium.

2. A process according to claim 1, characterised in that there is used a microorganism strain obtained by protoplast fusion between different strains of Corynebacterium or Brevibacterium and which has a resistance to two or more antibiotics.

3. A process according to claim 1 or 2, characterised in that there is used a microorganism strain having a resistance to two or more antibiotics, being antibiotics selected from aminoglycoside antibiotics, B-lactam type antibiotics, macrolide type antibiotics, peptide type antibiotics and rifamycin type antibiotics.

4. A process according to claim 3, characterised in that there is used a microorganism strain having a resistance to two or more of the following: penicillin G, cephalosporin C, streptomycin, dihydrostreptomycin, rifampicin, chloramphenicol, tetracycline, spiramycin, erythromycin, kanamycin, kasugamycin, polymixin, colistin, lincomycin, gentamicin, sagamicin, fortimicin, oleandomycin, bacitracin, gramicidin, thiocillin, bacilysin, polcillin, brevolin, surfactin, circulin, tyrocidin and subtilysin.

5. A process according to any one of claims 1-4, characterised in that the microorganism used in a mutant strain of, or is obtained by protoplast fusion of two different strains of, Corynebacterium glutamicum or Brevibacterium lactofermentum.

6.    A process according to claim 1, characterised in that said microorganism is the protoplast fusion strain identified by the deposit number FERM BP-417.

7.    A process according to claim 1, characterised in that said microorganism is Corynebacterium glutamicum FERM BP-414, Corynebacterium glutamicum FERM BP-418, Corynebacterium glutamicum FERM BP-427, Corynebacterium glutamicum FERM BP-416, Brevibacterium lactofermentum FERM BP-419 or Brevibacterium lactofermentum FERM BP-415

8.    A process according to any one of claims 1-7, characterised in that the culture is conducted at 20 to 40°C for a period of from 1 to 4 days.

9.    A microorganism having an ability to produce L-glutamic acid, characterised in that the microorganism has been obtained by the technology of protoplast fusion.

10.    A microorganism according to claim 9, characterised in that it has been obtained by protoplast fusion between the same or different strains of the genus Corynebacterium or Brevibacterium.

11.    A microorganism according to claim 10, characterised in that it has been obtained by protoplast fusion between the same or different strains belonging to the species of Corynebacterium glutamicum or Brevibacterium lactofermentum.

12.    A microorganism according to claim 9,10 or 11 characterised in that it has a resistance to two or more antibiotics.

13.    A microorganism according to claim 12, characterised in that it has resistance to two or more antibiotics selected from the group consisting of aminoglycoside type antibiotics, B-lactam type antibiotics, peptide type antibiotics, macrolide type antibiotics and rifamycin type antibiotics.

14. A microorganism according to claim 12, characterised in that it has resistance to two or more of the following: penicillin G, cephalosporin C, streptomycin, dihydrostreptomycin, rifampicin, chloramphenicol, tetracycline, spiramycin, erythromycin, kanamycin, kasugamycin, polymixin, colistin, lincomycin, gentamicin, sagamicin, fortimicin, oleandomycin, bacitracin, gramicidin, thiocillin, bacilysin, polcillin, brevolin, surfactin, circulin, tyrocidin and subtilysin.

15. A biologically pure culture of <u>Corynebacterium glutamicum</u> characterised in that it has the identifying characteristics of the microorganism strain identified by the deposit number FERM BP-414, FERM BP-418, FERM BP-416 or FERM BP-427.

16. A biologically pure culture of <u>Brevibacterium lactofermentum,</u> characterised in that it has the identifying characteristics of the microorganism strain identified by the deposit number FERM BP-415 or FERM BP-419.

17. A biologically pure culture of a protoplast fusion strain characterised in that it has the identifying characteristics of FERM BP-417.

18. A biologically pure culture of an L-glutamic acid producing microorganism of the genus <u>Corynebacterium</u> or <u>Brevibacterium,</u> characterised in that the microorganism has, or has been endowed with, a resistance to two or more antibiotics.

19. A microorganism according to claim 18, characterised by resistance to two or more antibiotics of the type specified in claim 13.

20. A microorganism according to claim 18, characterised by resistance to two or more of the antibiotics specified in claim 14.